# EUROPEAN PATENT APPLICATION

(11) **EP 4 306 528 A1**
(43) Date of publication of application: **17.01.2024**
(21) Application number: 22930459.7
(22) Date of filing: 17.05.2022
(51) Int. Cl.: C07D 519/00, A61K 31/519, A61P 35/00, A61P 29/00, A61P 37/02

(54) **HEMTAC SMALL MOLECULE DEGRADATION AGENT AND USE THEREOF**

(30) Priority: 11.03.2022 CN 202210241970
(71) Applicant: Shandong University, Jinan, Shandong 250012 (CN)
(72) Inventor: LI, Minyong, Jinan, Shandong 250012 (CN); DU, Lvpei, Jinan, Shandong 250012 (CN); LI, Zhenzhen, Jinan, Shandong 250012 (CN)
(74) Representative: Behr, Wolfgang
(86) International application number: PCT/CN2022/093374
(87) International publication number: WO 2023/168830

(57) **Abstract**

The present invention relates to the technical field of biological medicine, and particularly to a heat shock protein 90 (HSP90)-mediated targeting chimera (HEMTAC) small-molecule degradation agent and an application thereof. It is found through research in the present invention that the HEMTAC small-molecule degradation agent can induce formation of an HSP90-HEMTAC-targeted protein ternary complex, the targeted protein is spatially positioned at a position which is beneficial for being mistakenly recognized as an "abnormal" protein by the HSP90, is then introduced into a ubiquitin-proteasome system, and causes degradation of the targeted protein. HEMTACs are released to participate in the next degradation cycle. Therefore, the HEMTACs have a catalytic effect in targeted protein ubiquitin induction and degradation, can be used for treating diseases related to tumors, inflammations, immunity and the like, and have a good prospect for practical application.

## Description

### TECHNICAL FIELD

The present invention relates to the technical field of biological medicine, and particularly to a heat shock protein 90 (HSP90)-mediated targeting chimera (HEMTAC) small-molecule degradation agent and an application thereof.

### BACKGROUND

Information disclosed in the background section is merely for the purpose of facilitating the understanding of the general background of the present invention and is not necessarily to be taken as an acknowledgment or any form of suggestion that the information constitutes prior art that is already known to those of ordinary skill in the art.

In the continuous exploration of pharmaceutical chemistry, new strategies for targeting disease kinesins are constantly being developed. The targeted protein degradation strategy induced by small molecules is the most noteworthy strategy in recent years. This strategy can target inactive sites or undruggable protein targets, making it possible to degrade any protein. At present, the main degradation technologies are proteolysis-targeting chimeras (PROTACs), lysosome-targeting chimeras (LYTACs) and antibody-based PROTACs (AbTACs). The candidate drugs ARV-110 and ARV-471 related to the PROTAC technology have entered the phase II clinical trial. The PROTAC technology is mainly suitable for intracellular proteins, while the LYTAC and AbTAC technologies can degrade exocrine proteins and membrane proteins. Protein degradation technologies have shown great potential in targeting undruggable targets and disease treatment.

Although the emergence of these novel degradation technologies can overcome the limitations of small molecule inhibitors, the emerging degradation technologies also have some limitations, which may lead to the degradation of targeted proteins that are not involved in the disease process in many tissues and organs, resulting in serious side effects in the treatment process. In addition, small molecule PROTAC plays its degradation role by "hijacking" E3 ligase. However, studies have shown that small molecule PROTAC based on VHL and CRBN can cause an off-target effect and drug resistant mechanism in cells. Therefore, it is urgent to expand the scope of targeted degradation technology and develop novel protein degradation technologies.

The heat shock protein 90 (HSP90) family is an ATP-dependent chaperone with a molecular weight of about 90 kDa. HSP90 exists in cells in the form of dimers. The dimerization of HSP90 is necessary for its intracellular function. The expression of HSP90 in the non-stressed state accounts for about 1% to 2% of the total intracellular protein, and is thousands of times the average content of protein. Under stress conditions, the content of HSP90 can increase to 4% to 6% of the total intracellular protein. In human cells, HSP90 protein is classified into HSP90α (inducible type) and HSP90β (constitutive type). The expression of HSP90α increases after heat induction, and is related to the maintenance of cell homeostasis under pressure. HSP90β can be expressed continuously, is indispensable in mammals, and is related to the life activities of mammals.

As an important molecular chaperone, HSP90 can activate different substrate proteins, and therefore participate in the regulation of various life activities. In order to make proteins perform their normal biological functions, molecular chaperones can activate newly synthesized proteins, assemble and depolymerize molecular complexes, help abnormally folded proteins refold, and coordinate with the ubiquitin-proteasome system to regulate and degrade misfolded proteins. In addition, it is found through research that heat shock protein family is closely related to tumors, the expression of HSP90 is abnormally increased in tumor cells induced by some oncogenes and their products, and HSP90 participates in tumor growth, invasion, and metastasis.

### SUMMARY

An objective of the present invention is to provide a HEMTAC small molecule degradation agent for inducing targeted protein degradation based on HSP90 and an application thereof. According to the characteristics that HSP90 has stable client protein, can degrade error protein by the ubiquitin-proteasome system, and has high expression in tumor cells, the present invention designs and develops a novel protein degradation technology, which is named as heat shock protein 90 (HSP90)-mediated targeting chimeras (HEMTACs). Based on the above research results, the present invention is attained.

The following technical solution is adopted in the present invention.

According to a first aspect of the present invention, a compound is provided, having a structure of formula (I) below:

R₁-L-R₂ formula (I)

wherein:
(a) R1 is a ligand/conjugate of a targeted protein;
(b) R2 is an HSP90 ligand; and
(c) L is a linker through which R1 and R2 are linked.

The above compound is actually a HEMTACs small molecule degradation agent.

The small molecule HEMTACs includes three parts: a ligand bound to the targeted protein, a linker, and a ligand bound to HSP90. The linker connects the two ligands to form the small molecule HEMTACs. Similar to the PROTAC technology, the small molecule HEMTACs can induce formation of an HSP90-HEMTAC-targeted protein ternary complex, the targeted protein is spatially positioned at a position which is beneficial for being mistakenly recognized as an "abnormal" protein by the HSP90, is then introduced into a ubiquitin-proteasome system, and causes degradation of the targeted protein. HEMTACs are released to participate in the next degradation cycle. Therefore, the small molecule HEMTACs have a catalytic effect in targeted protein ubiquitin induction and degradation.

The targeted protein includes, but is not limited to, kinases, a G protein-coupled receptor, a transcription factor, phosphatase, and a member of the RAS superfamily.

Therefore, R1 is selected from compounds targeting the following: kinases, a G protein-coupled receptor, a transcription factor, phosphatase, and a member of the RAS superfamily.

Specifically, R1 is Palbociclib.

R2 is a ligand BIIB021 of HSP90 or a derivative thereof.

The compound further includes pharmaceutically acceptable salts, stereoisomers, esters, prodrugs, solvates, and deuterated compounds thereof.

According to a second aspect of the present invention, an application of the compound in preparing drugs for tumors, inflammation, and immune-related diseases.

Further, the diseases may also be diseases associated with cell cycle dependent protein kinases 4 and 6 (CDK4/6).

According to a third aspect of the present invention, a pharmaceutical composition is provided, including the above compound.

According to a fourth aspect of the present invention, a pharmaceutical preparation is provided, including the above compound and at least one pharmaceutically acceptable pharmaceutically inactive ingredient.

It is obvious that the pharmaceutical composition or pharmaceutical preparation can be used for the treatment of tumors, inflammation, immune-related diseases, especially, diseases associated with CDK4/6.

According to a fifth aspect of the present invention, a method of treating tumors, inflammation, and immune-related disease is provided. The method includes administering to a subject a therapeutically effective amount of the above compound, pharmaceutical composition, or pharmaceutical preparation.

### The one or more technical solutions described above have the following technical effects:

The above technical solutions report for the first time a HEMTAC small molecule degradation agent for inducing targeted protein degradation based on HSP90 and an application thereof. It is found through research in the present invention that the HEMTAC small-molecule degradation agent can induce formation of an HSP90-HEMTAC-targeted protein ternary complex, the targeted protein is spatially positioned at a position which is beneficial for being mistakenly recognized as an "abnormal" protein by the HSP90, is then introduced into a ubiquitin-proteasome system, and causes degradation of the targeted protein. HEMTACs are released to participate in the next degradation cycle. Therefore, the HEMTACs have a catalytic effect in targeted protein ubiquitin induction and degradation, can be used for treating diseases related to tumors, inflammations, immunity and the like, and have a good prospect for practical application.

### BRIEF DESCRIPTION OF THE DRAWINGS

The drawings forming a part of the present invention are used to provide further understanding of the present invention, and the exemplary embodiments and description of the present invention are used to explain the present invention but do not constitute an improper limitation on the present invention.

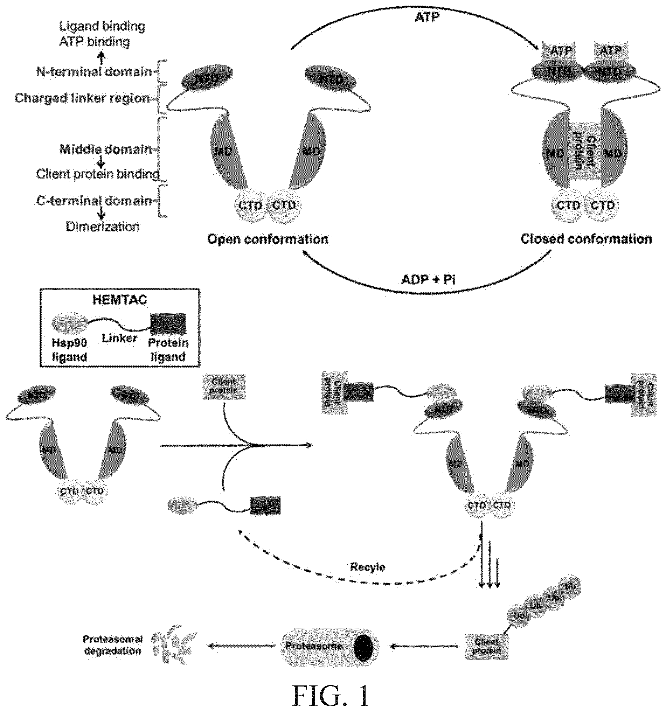

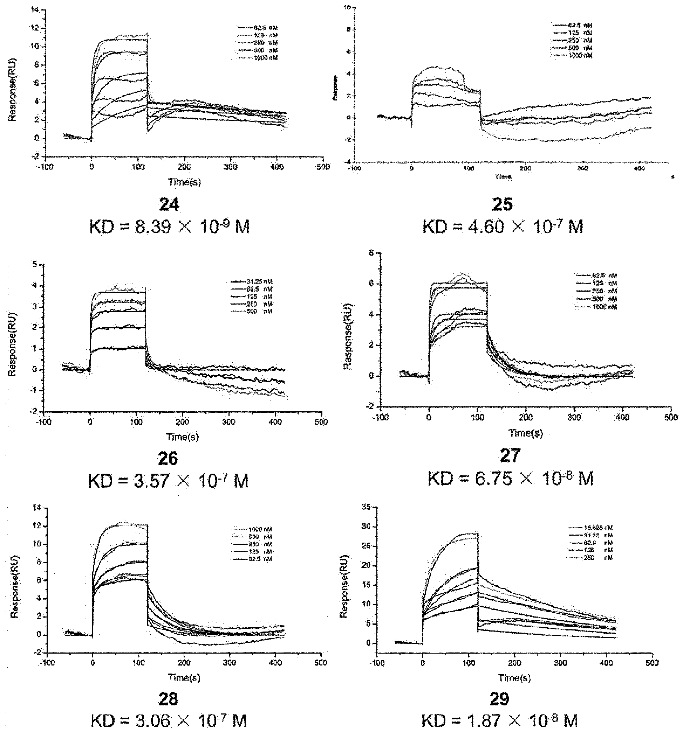

FIG. 1 is a schematic diagram showing functions of HSP90 molecular chaperone and a schematic diagram of protein degradation mechanism of small molecule HEMTACs.
FIG. 2 shows the binding affinity of compounds to HSP90α determined by surface plasmon resonance (SPR).
FIG. 3 shows the effect of HEMTAC 26 at different concentrations and time on CDK4 and CDK6 protein levels in B16F10 cells.
FIG. 4 shows the anti-tumor activity of small molecule HEMTAC 26 in a mouse B16F10 melanoma model, where the experimental results show that small molecule HEMTAC 26 has a significant anti-melanoma effect in vivo.

### DETAILED DESCRIPTION

It should be noted that the following detailed description is exemplary and is intended to provide a further description of the present invention. All technical and scientific terms used herein have the same meaning as commonly understood by one of ordinary skill in the art to which this invention belongs, unless otherwise indicated.

It is to be noted that the terminology used herein is for the purpose of describing particular embodiments, and is not intended to limit the exemplary embodiments of the present invention. As used herein, the singular terms are also intended to include the plural, and it is also to be understood that when the terms "include" and/or "comprise" are used in the specification, they indicate the presence of features, steps, operations, devices, components, and/or combinations thereof, unless otherwise indicated.

The present invention will be described in further detail with reference to specific examples. The following examples are illustrative of the present invention and the present invention is not limited thereto. Where no specific experimental conditions are given in the examples, conventional conditions or conditions recommended by reagent manufacturers are followed. The reagents and materials used in examples below are all commercially available, unless it is otherwise stated.

In a typical exemplary embodiment of the present invention, a compound is provided, having a structure of formula (I) below:

R₁-L-R₂ formula (I)

wherein:
(a) R1 is a ligand/conjugate of a targeted protein;
(b) R2 is an HSP90 ligand; and
(c) L is a linker through which R1 and R2 are linked.

The above compound is actually a HEMTACs small molecule degradation agent. The small molecule HEMTACs includes three parts: a ligand bound to the targeted protein, a linker, and a ligand bound to HSP90. The linker connects the two ligands to form the small molecule HEMTACs. Similar to the PROTAC technology, the small molecule HEMTACs can induce formation of an HSP90-HEMTAC-targeted protein ternary complex, the targeted protein is spatially positioned at a position which is beneficial for being mistakenly recognized as an "abnormal" protein by the HSP90, is then introduced into a ubiquitin-proteasome system, and causes degradation of the targeted protein. HEMTACs are released to participate in the next degradation cycle. Therefore, the small molecule HEMTACs have a catalytic effect in targeted protein ubiquitin induction and degradation.

The targeted protein includes, but is not limited to, kinases, a G protein-coupled receptor, a transcription factor, phosphatase, and a member of the RAS superfamily.

Therefore, R1 is selected from compounds targeting the following: kinases, a G protein-coupled receptor, a transcription factor, phosphatase, and a member of the RAS superfamily.

Specifically, R1 is Palbociclib.

R2 is a ligand BIIB021 of HSP90 or a derivative thereof.

The general formula of the ligand BIIB021 of HSP90 or the derivative thereof is as follows:

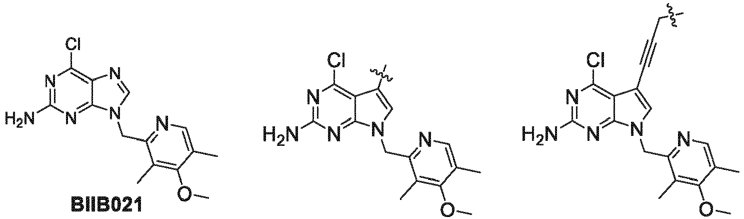

In another exemplary embodiment of the present invention, the compound includes any one or more of:

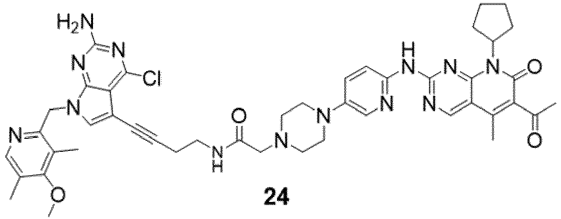

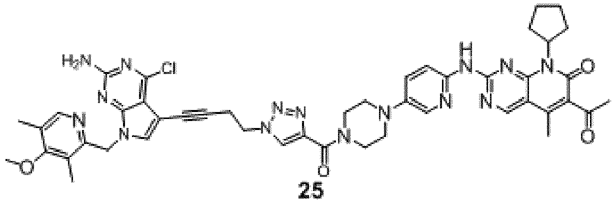

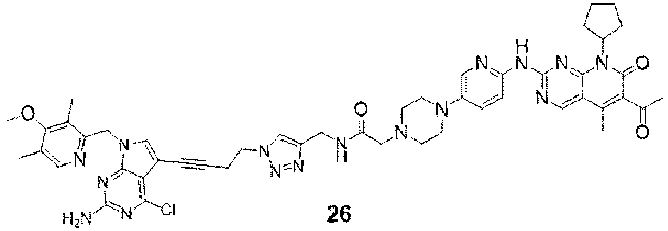

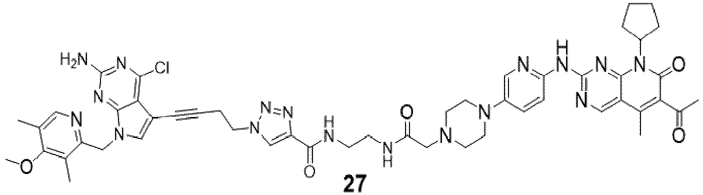

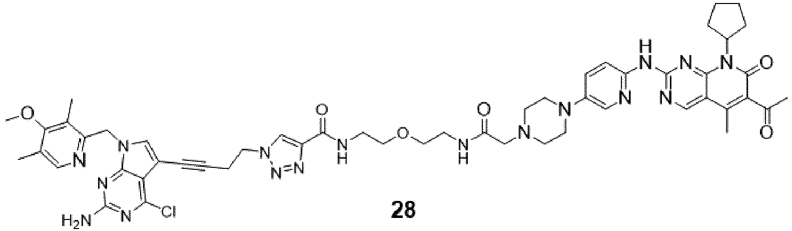

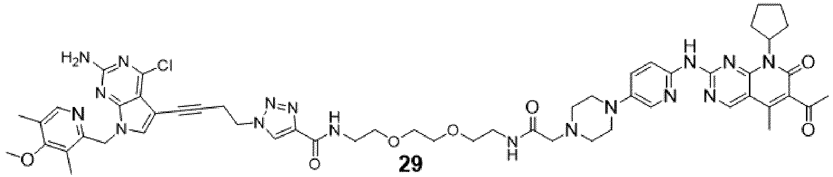

In another exemplary embodiment of the present invention, the compound further includes pharmaceutically acceptable salts, stereoisomers, esters, prodrugs, solvates, and deuterated compounds thereof.

In another exemplary embodiment of the present invention, an application of the compound in preparing drugs for tumors, inflammation, and immune-related diseases.

In another exemplary embodiment of the present invention, the diseases may also be diseases associated with cell cycle dependent protein kinases 4 and 6 (CDK4/6).

In another exemplary embodiment of the present invention, a pharmaceutical composition is provided, including the above compound. The pharmaceutical composition can be used alone or in combination with other kinds of drugs for treating diseases such as tumors, inflammation, immunity, etc.

In another exemplary embodiment of the present invention, a pharmaceutical preparation is provided, including the above compound and at least one pharmaceutically acceptable pharmaceutically inactive ingredient.

The pharmaceutically inactive ingredient can be a carrier, an excipient, a diluent, etc. commonly used in pharmacy. Moreover, according to commonly used methods, the pharmaceutical preparation can be prepared into oral preparations such as powder, granule, tablet, capsule, suspension, emulsion, syrup and spray, external preparations, suppositories and sterile injection solutions for use.

The pharmaceutically inactive ingredient such as a carrier, an excipient, and a diluent that may be contained is well known in the art and can be determined by those of ordinary skill in the art to meet clinical standards.

In another exemplary embodiment of the present invention, the carrier, excipient and diluent include, but are not limited to, lactose, glucose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc powder, magnesium stearate and mineral oil.

In another exemplary embodiment of the present invention, the pharmaceutical preparation of the present invention can be administered to a body in a known manner. For example, the pharmaceutical preparation is delivered to a tissue of interest by intravenous systemic delivery or local injection. Optionally, the pharmaceutical preparation is administered via intravenous, percutaneous, intranasal, mucosal, or other means of delivery. Such administration can be performed via a single dose or multiple doses. It will be understood by those skilled in the art that the actual dose to be administered in the present invention may vary to a large extent depending on a number of factors, such as the target cell, the biological type or tissue thereof, the general condition of the subject to be treated, the route of administration, the mode of administration, and the like.

In another exemplary embodiment of the present invention, the pharmaceutical preparation can be administered to humans and non-human mammals, such as mice, rats, guinea pigs, rabbits, dogs, monkeys, orangutans, etc.

It is obvious that the pharmaceutical composition or pharmaceutical preparation can be used for the treatment of tumors, inflammation, immune-related diseases, especially, diseases associated with CDK4/6.

According to a fifth aspect of the present invention, a method of treating tumors, inflammation, and immune-related disease is provided. The method includes administering to a subject a therapeutically effective amount of the above compound, pharmaceutical composition, or pharmaceutical preparation.

The subject refers to an animal, preferably a mammal, most preferably a human, which has been the subject of treatment, observation or experiment.

The "therapeutically effective amount" refers to an amount of an active compound or agent, including the compound of the present invention, that elicits a biological or medical response of the tissue system, animal or human that is expected by a researcher, veterinarian, physician or other medical personnel. The biological or medical response includes alleviating or partially alleviating the symptoms of the disease, syndrome, disorder or condition under treatment.

Researchers, veterinarians, physicians or other medical personnel in the art may learn of the range of the therapeutically effective amount available from clinical trials or other means well known in the art.

The present invention is further illustrated through examples below; however the present invention is not limited thereto. It is to be understood that these examples are merely illustrative of the present invention and are not intended to limit the scope of the present invention. In examples below where no specific conditions are given in the experimental methods, conventional conditions are usually followed.

### Example 1: Preparation of Compound 24

### Synthesis of Compound 2

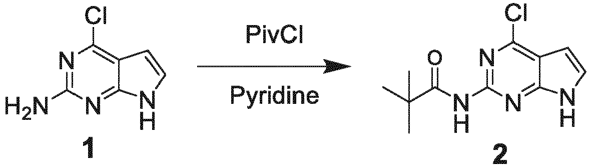

Compound 1 (2 g, 11.86 mmol, 1.0 equiv.) was dissolved in 20 mL of pyridine. Then trimethyl acetyl chloride (4.45 mL, 35.59 mmol, 3 equiv.) was added and stirred overnight at room temperature to give a mixture of N(2) monoacylation and N(2),N(7) diacylation. The solvent was rotary dried. The residue was dissolved in a mixture of ammonia (25% NH₃, 5 mL) and methanol (20 mL), and stirred at room temperature for 30 minutes, to selectively disconnect the neovaleryl group at N(7) position. The solid was filtered. The filter cake was washed with water (10 mL×3), and vacuum dried to obtain 2.55 g of Compound 2, yellow solid, with a yield of 85%. ¹H NMR (400 MHz, DMSO-*d₆*) δ 12.32 (s, 1H), 10.01 (s, 1H), 7.54 (d, J = 3.6 Hz, 1H), 6.53 (d, J = 3.5 Hz, 1H), 1.24 (s, 9H). ESI-MS: *m*/*z* [M+H]⁺ calculated for C₁₁H₁₄ClN₄O⁺ 253.09, found 253.06.

### Synthesis of Compound 3

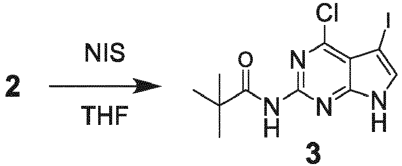

Compound 2 (200 mg, 791.45 µmol, 1.0 equiv.) was dissolved in dry tetrahydrofuran (10 mL). Then N-iodosuccinimide (213.68 mg, 949.74 µmol, 1.2 equiv.) was added under the protection of N₂. he mixture was reacted at room temperature for 1 hour. The solvent was rotary dried. The residue was dissolved in dichloromethane (60 mL), and washed with saturated Na₂S₂O₃ solution (40 mL×3) and brine (30 mL×3). The organic layer was dried over anhydrous Na₂SO₄. The organic solvent was removed under reduced pressure. The crude product was purified by silica gel column chromatography (dichloromethane/methanol = 60:1 to 30: 1) to obtain 260 mg of Compound 3, brown solid, with a yield of 87%. ¹H NMR (400 MHz, DMSO-*d₆*) δ 12.69 (s, 1H), 10.09 (s, 1H), 7.77 (d, *J =* 1.2 Hz, 1H), 1.23 (s, 9H). ESI-MS: *m*/*z* [M-H]⁻ calculated for C₁₁H₁₁ClIN₄O⁻ 376.97, found 377.08.

### Synthesis of Compound 5

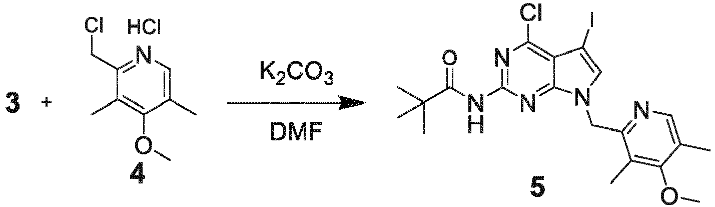

Compound 3 (255 mg, 673.54 µmol, 1.0 equiv.), Compound 4 (157.08 mg, 707.21 µmol, 1.1 equiv.) and K₂CO₃ (279.26 mg, 2.02 mmol, 3.0 equiv.) were dissolved in dry DMF (5 mL) and stirred at room temperature for 24 hours. The reaction solution was added dropwise to a mixed solution of water (15 mL) and isopropanol (15 mL). The solid was filtered. The filter cake was washed with water (15 mL), and vacuum dried to obtain 323 mg of Compound 5, white solid, with a yield of 91%. ¹H NMR (400 MHz, DMSO-*d₆*) δ 10.14 (s, 1H), 8.05 (s, 1H), 7.73 (s, 1H), 5.46 (s, 2H), 3.74 (s, 3H), 2.33 (s, 3H), 2.16 (s, 3H), 1.21 (s, 9H). ESI-MS: *m*/*z* [M+H]⁺ calculated for C₂₀H₂₄ClIN₅O₂⁺ 528.07, found 528.31.

### Synthesis of Compound 6

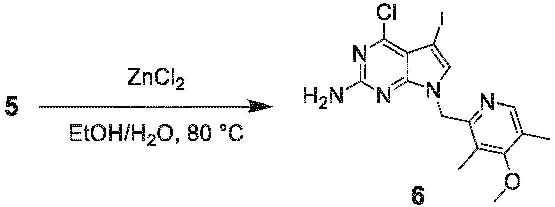

Compound 5 (2.0 g, 3.79 mmol, 1.0 equiv.) was dissolved in a mixed solvent of ethanol/water (20:1, 42 mL). Then ZnCl₂ (2.58 g, 18.95 mmol, 5.0 equiv.) was added to the reaction solution, heated to 80 °C and stirred for 20 hours. The reaction solution was cooled to room temperature, and poured into water (50 mL). The solid was filtered. The filter cake was washed with water (10 mL×3), and vacuum dried to obtain 1.44 g of Compound 6, white solid, with a yield of 86%. ¹H NMR (400 MHz, DMSO-*d₆*) δ 8.07 (s, 1H), 7.26 (s, 1H), 6.71 (s, 2H), 5.29 (s, 2H), 3.73 (s, 3H), 2.26 (s, 3H), 2.17 (s, 3H). ESI-MS: *m*/*z* [M+H]⁺ calculated for C₁₅H₁₆ClIN₅O⁺ 444.01, found 444.11.

### Synthesis of Compound 7

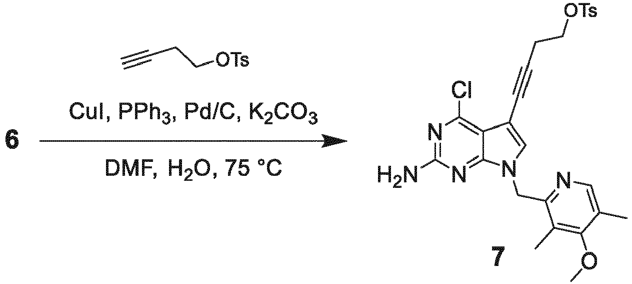

Compound 6 (1.0 g, 2.25 mmol, 1.0 equiv.), CuI (42.93 mg, 225.39 µmol, 0.1 equiv.), PPh₃ (59.12 mg, 225.39 µmol, 0.1 equiv.), 10% Pd/C (66.73 mg, 56.35 µmol, 0.025 equiv.), and K₂CO₃ (342.65 mg, 2.48 mmol, 1.1 equiv.) were dissolved in a mixed solvent of DMF (12 mL) and water (4 mL). 3-butynyl toluenesulfonate dissolved in DMF (4 mL) was added dropwise to the reaction solution under the protection of N₂. The reaction solution was heated to 75°C and stirred for 3 hours. The reaction solution was filtered with diatomite. The diatomite was washed with ethyl acetate. Then the filtrate was diluted with 100 mL of ethyl acetate. The organic phase was washed with water (40 mLx3) and brine (40 mLx3) and dried over anhydrous Na₂SO₄. The organic solvent was removed under reduced pressure. The crude product was purified by silica gel column chromatography (petroleum ether/ethyl acetate = 1:1 to 1:3) to obtain 700 mg of Compound 7, white solid, with a yield of 74%. ¹H NMR (400 MHz, DMSO-*d₆*) δ 8.10 (s, 1H), 7.80 (d, *J* = 7.7 Hz, 2H), 7.41 (d, *J =* 7.6 Hz, 2H), 7.28 (s, 1H), 6.75 (s, 2H), 5.31 (s, 2H), 4.14 (t, *J=* 5.1 Hz, 2H), 3.73 (s, 3H), 2.77 (t, *J=* 5.1 Hz, 2H), 2.33 (s, 3H), 2.27 (s, 3H), 2.17 (s, 3H). ESI-MS: *m*/*z* [M+H]⁺ calculated for C₂₆H₂₇ClN₅O₄S⁺ 540.15, found 540.13.

### Synthesis of Compound 8

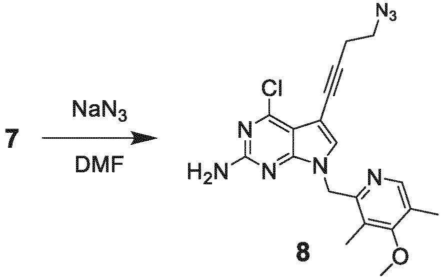

Compound 7 (406 mg, 751.80 µmol, 1.0 equiv.) was dissolved in DMF (8 mL). Then NaN₃ (146.63 mg, 2.26 mmol, 3.0 equiv.) was added to the reaction solution. The reaction solution was heated to 60°C and stirred for 4 hours. After cooling to room temperature, the reaction solution was poured into water, and extracted with ethyl acetate three times (40 mL×3). The organic phases were combined, washed with water and brine, and dried over anhydrous Na₂SO₄. The organic solvent was removed under reduced pressure. The crude product was purified by silica gel column chromatography (petroleum ether/ethyl acetate = 1:1 to 1:3) to obtain 290 mg of Compound 8, white solid, with a yield of 94%. ¹H NMR (400 MHz, DMSO-*d₆*) δ 8.06 (s, 1H), 7.28 (s, 1H), 6.71 (s, 2H), 5.29 (s, 2H), 3.73 (s, 3H), 3.51 (t, *J =* 6.5 Hz, 2H), 2.72 (t, *J =* 6.5 Hz, 2H), 2.25 (s, 3H), 2.17 (s, 3H). ESI-MS: m/z [M+H]⁺ calculated for C₁₉H₂₀ClN₈O⁺ 411.14, found 411.03.

### Synthesis of Compound 9

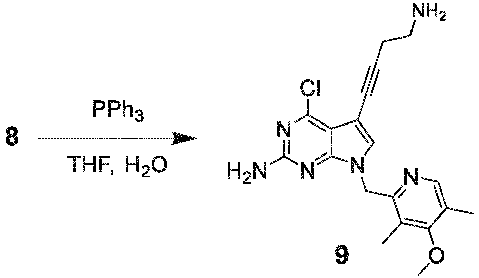

Compound 8 (810 mg, 1.97 mmol, 1.0 equiv.) was dissolved in a mixed solvent of tetrahydrofuran (30 mL) and water (2 mL). Then PPh₃ (1.55 g, 5.91 mmol, 3.0 equiv.) was add to the reaction solution, and stirred overnight at room temperature. The solvent was rotary dried. The crude product was purified by silica gel column chromatography (dichloromethane/methanol = 30:1 to 5:1) to obtain 480 mg of Compound 9, white solid, with a yield of 63%. ¹H NMR (400 MHz, DMSO-*d₆*) δ 8.06 (s, 1H), 7.30 (s, 1H), 6.73 (s, 2H), 5.28 (s, 2H), 3.73 (s, 3H), 2.92 (t, *J =* 6.9 Hz, 2H), 2.65 (t, *J=* 7.0 Hz, 2H), 2.25 (s, 3H), 2.16 (s, 3H), 1.23 (s, 2H). ESI-MS: *m*/*z* [M+H]⁺ calculated for C₁₉H₂₂ClN₆O⁺ 385.15, found 385.10.

### Synthesis of Compound 11

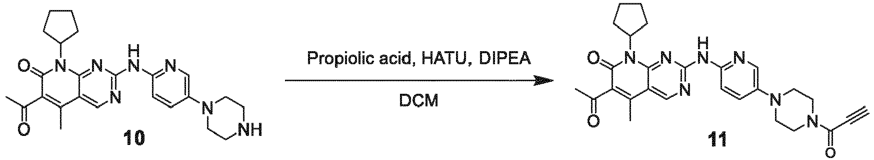

Compound 10 (150 mg, 335.16 µmol, 1.0 equiv.) and propargic acid (30.95 µL, 502.74 µmol, 1.5 equiv.) were dissolved in dichloromethane (15 mL). Then HATU (156.05 mg, 402.20 µmol, 1.2 equiv.) and DIPEA (169.58 µL, 1.01 mmol, 3.0 equiv.) were added to the suspension, and stirred at room temperature for 3 hours. The reaction solution was diluted with 100 mL of dichloromethane, and washed with water and brine. The organic phases were dried over anhydrous Na₂SO₄. The organic solvent was removed under reduced pressure. The crude product was purified by silica gel column chromatography (dichloromethane/methanol = 70:1 to 40:1) to obtain 105 mg of Compound 11, yellow solid, with a yield of 63%. ¹H NMR (400 MHz, DMSO-*d₆*) δ 10.15 (s, 1H), 8.96 (s, 1H), 8.09 (d, *J* = 2.7 Hz, 1H), 7.90 (d, *J* = 9.0 Hz, 1H), 7.52 (dd, *J* = 9.1, 2.7 Hz, 1H), 5.94 - 5.77 (m, 1H), 4.62 (s, 1H), 3.96 - 3.79 (m, 2H), 3.75 - 3.60 (m, 2H), 3.32 - 3.20 (m, 2H), 3.21 - 3.06 (m, 2H), 2.43 (s, 3H), 2.31 (s, 3H), 2.29 - 2.14 (m, 2H), 2.00 - 1.84 (m, 2H), 1.85 - 1.69 (m, 2H), 1.68 - 1.50 (m, 2H). ESI-MS: *m*/*z* [M+H]⁺ calculated for C₂₇H₃₀N₇O₃⁺ 500.24, found 500.17.

### Synthesis of Compound 12

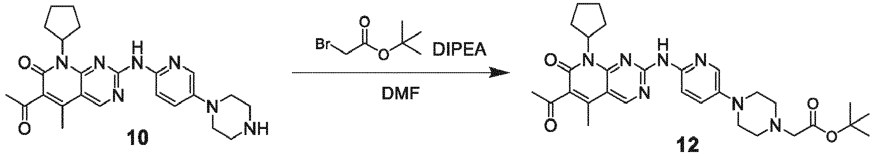

Compound 10 (1.0 g, 2.23 mmol, 1.0 equiv.) was dissolved in DMF (15 mL). Then tert-butyl bromoacetate (398.86 µL, 2.68 mmol, 1.2 equiv.) and DIPEA (1.13 mL, 6.70 mmol, 3.0 equiv.) were successively added to the reaction solution, and stirred overnight at room temperature. Then the reaction solution was poured into water (50 mL). The solid was filtered. The filter cake was washed with water (30 mL), and vacuum dried to obtain 1.2 g of Compound 12, yellow solid, with a yield of 95%. ¹H NMR (400 MHz, CDCl₃) δ 8.82 (s, 1H), 8.23 (s, 1H), 8.15 (d, *J =* 9.1 Hz, 1H), 8.06 (d, *J =* 2.8 Hz, 1H), 7.32 (dd, *J =* 9.1, 2.9 Hz, 1H), 5.88 (p, *J =* 8.9 Hz, 1H), 3.34 - 3.22 (m, 4H), 3.20 (s, 2H), 2.92 - 2.64 (m, 4H), 2.55 (s, 3H), 2.41 - 2.30 (m, 5H), 2.11 - 2.02 (m, 2H), 1.92 - 1.84 (m, 2H), 1.74 - 1.67 (m, 2H), 1.49 (s, 9H). ESI-MS: *m*/*z* [M+H]⁺ calculated for C₃₀H₄₀N₇O₄⁺ 562.31, found 562.25.

### Synthesis of Compound 13

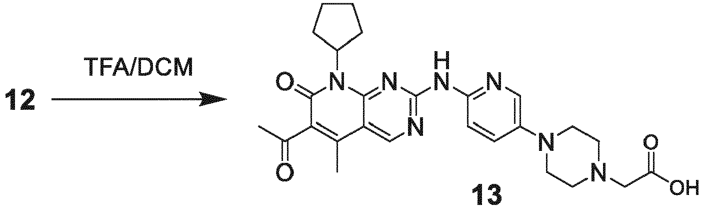

Compound 12 (1.0 g, 1.78 mmol, 1.0 equiv.) was dissolved in a mixed solvent of trifluoroacetic acid (5 mL) and dichloromethane (5 mL) and stirred at room temperature for 2 hours. Most of the solvent in the reaction solution was rotary dried. A small amount of toluene was added and then rotary dried. This operation was repeated many times until the residual trifluoroacetic acid was removed. The crude product was directly used for the next reaction without further purification.

### Synthesis of Compound 14

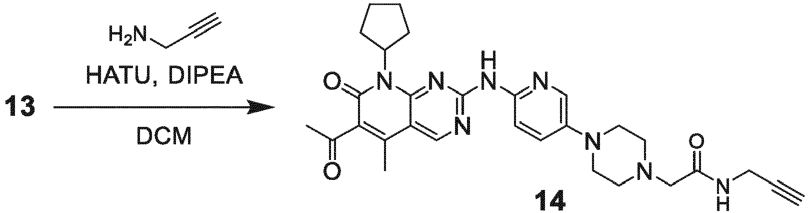

Compound 13 (255 mg, 504.37 µmol, 1.0 equiv.) was dissolved in dry dichloromethane (20 mL). Then propargyl amine (49.44 µL, 756.56 µmol, 1.5 equiv.), HATU (287.67 mg, 757.5 µmol, 1.5 equiv.) and DIPEA (448.24 µ l, 2.52 mmol, 5.0 equiv.) were successively added to the reaction solution, and stirred at room temperature for 5 hours. The reaction solution was diluted with 100 mL of dichloromethane, and washed with water and brine. The organic phases were dried over anhydrous Na₂SO₄. The organic solvent was removed under reduced pressure. The crude product was purified by silica gel column chromatography (dichloromethane/methanol = 60:1 to 30:1) to obtain 185 mg of Compound 14, yellow solid, with a yield of 68%. ¹H NMR (400 MHz, DMSO-*d₆*) δ 10.09 (s, 1H), 8.95 (s, 1H), 8.20 (t, J = 5.7 Hz, 1H), 8.06 (d, *J =* 2.7 Hz, 1H), 7.85 (d, *J* = 9.0 Hz, 1H), 7.48 (dd, *J =* 9.1, 2.8 Hz, 1H), 5.89 - 5.76 (m, 1H), 3.89 (dd, *J=* 5.7, 2.3 Hz, 2H), 3.21 (t, 4H), 3.07 (t, *J=* 2.3 Hz, 1H), 3.02 (s, 2H), 2.60 (t, 4H), 2.42 (s, 3H), 2.31 (s, 3H), 2.28 - 2.14 (m, 2H), 1.97 - 1.82 (m, 2H), 1.82 - 1.68 (m, 2H), 1.65 - 1.50 (m, 2H). ESI-MS: *m*/*z* [M+H]⁺ calculated for C₂₉H₃₅N₈O₃⁺ 543.28, found 543.22.

### Synthesis of Compound 15

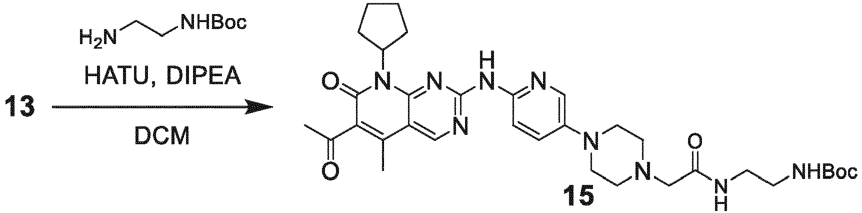

Compound 13 (257 mg, 508.33 µmol, 1.0 equiv.) was dissolved in dry dichloromethane (20 mL). Then N-tert-butoxycarbonyl-1, 2-ethylenediamine (97.73 mg, 609.99 µmol, 1.2 equiv.), HATU (231.94 mg, 609.99 µmol, 1.2 equiv.) and DIPEA (451.76 µL, 2.54 mmol, 5.0 equiv.) were successively added to the reaction solution, and stirred overnight at room temperature. The reaction solution was diluted with 100 mL of dichloromethane, and washed with water and brine. The organic phases were dried over anhydrous Na₂SO₄. The organic solvent was removed under reduced pressure. The crude product was purified by silica gel column chromatography (dichloromethane/methanol = 60:1 to 30:1) to obtain 243 mg of Compound 15, yellow solid, with a yield of 74%. ¹H NMR (400 MHz, DMSO-*d₆*) δ 10.10 (s, 1H), 8.96 (s, 1H), 8.06 (d, *J =* 2.8 Hz, 1H), 7.96 - 7.73 (m, 2H), 7.47 (dd, *J =* 9.1, 2.8 Hz, 1H), 6.86 (t, *J =* 5.3 Hz, 1H), 5.88 - 5.77 (m, 1H), 3.29 - 3.11 (m, 6H), 3.08 - 2.90 (m, 4H), 2.61 (s, 4H), 2.43 (s, 3H), 2.32 (s, 3H), 2.29 - 2.18 (m, 2H), 1.95 - 1.84 (m, 2H), 1.83 - 1.72 (m, 2H), 1.64 - 1.55 (m, 2H), 1.38 (s, 9H). ESI-MS: *m*/*z* [M+H]⁺ calculated for C₃₃H₄₆N₉O₅⁺ 648.36, found 648.18.

### Synthesis of Compound 16

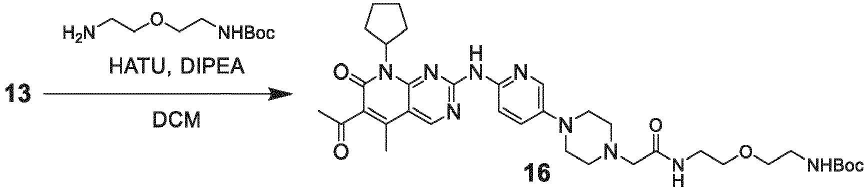

According to the synthesis method of Compound 15, Compound 13 (166.52 mg, 329.36 µmol, 1.0 equiv.) and tert-butyl 2-(2-(2-aminoethoxy)ethoxy)ethylcarbamate (82.38 mg, 395.24 µmol, 1.2 equiv.) were used as raw materials to obtain 150 mg of Compound 16, yellow solid, with a yield of 66%. ¹H NMR (400 MHz, DMSO-*d₆*) δ 10.10 (s, 1H), 8.96 (s, 1H), 8.07 (d, *J =* 2.6 Hz, 1H), 7.85 (d, *J =* 9.1 Hz, 1H), 7.80 (s, 1H), 7.48 (dd, J= 9.0, 2.6 Hz, 1H), 6.77 (t, *J =* 4.9 Hz, 1H), 5.87 - 5.75 (m, 1H), 3.48 - 3.38 (m, 4H), 3.31 - 3.18 (m, 6H), 3.11 - 2.94 (m, 4H), 2.63 (s, 4H), 2.43 (s, 3H), 2.31 (s, 3H), 2.29 - 2.18 (m, 2H), 1.95 - 1.83 (m, 2H), 1.82 - 1.70 (m, 2H), 1.65 - 1.53 (m, 2H), 1.37 (s, 9H). ESI-MS: *m*/*z* [M+H]⁺ calculated for C₃₅H₅₀N₉O₆⁺ 692.39, found 692.21.

### Synthesis of Compound 17

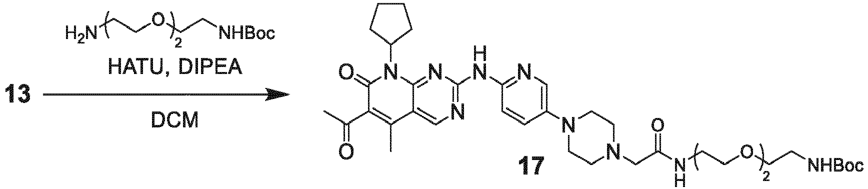

According to the synthesis method of Compound 15, Compound 13 (300 mg, 593.38 µmol, 1.0 equiv.) and tert-butyl [2-(2-aminoethoxy)ethoxy]ethylcarbamate (176.82 mg, 712.06 µmol, 1.2 equiv.) were used as raw materials to obtain 310 mg of Compound 17, yellow solid, with a yield of 71%. ¹H NMR (400 MHz, DMSO-*d₆*) δ 10.09 (s, 1H), 8.95 (s, 1H), 8.06 (d, *J=* 2.6 Hz, 1H), 7.85 (d, *J =* 9.0 Hz, 1H), 7.77 (s, 1H), 7.48 (dd, *J =* 9.1, 2.7 Hz, 1H), 6.74 (t, *J=* 5.4 Hz, 1H), 5.87 - 5.77 (m, 1H), 3.50 (s, 4H), 3.45 (t, *J =* 5.8 Hz, 2H), 3.39 - 3.36 (m, 2H), 3.30 - 3.26 (m, 2H), 3.21 (s, 3H), 3.12 - 2.81 (m, 6H), 2.62 (s, 3H), 2.42 (s, 3H), 2.31 (s, 3H), 2.28 - 2.18 (m, 2H), 1.93 - 1.83 (m, 2H), 1.81 - 1.72 (m, 2H), 1.64 - 1.54 (m, 2H), 1.36 (s, 9H). ESI-MS: *m*/*z* [M+H]⁺ calculated for C₃₇H₅₄N₉O₇⁺ 736.41, found 736.10.

### Synthesis of Compound 21

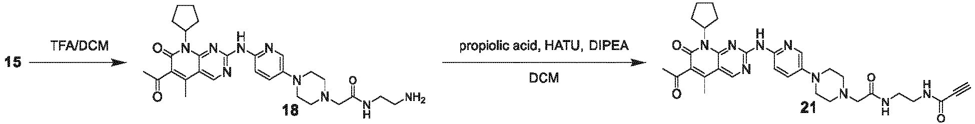

Compound 15 (245 mg, 378.21 µmol, 1.0 equiv.) was dissolved in a mixed solvent of dichloromethane (3 mL) and trifluoroacetic acid (3 mL) and stirred at room temperature for 30 minutes. Most of the solvent in the reaction solution was then rotary dried. A small amount of toluene was added and then rotary dried. This operation was repeated many times until the residual trifluoroacetic acid was removed. The obtained crude product of Compound 18 was directly used for the next reaction without further purification. The residue was dissolved in dichloromethane (15 mL). Then DIPEA (312.54 µL, 1.89 mmol, 5.0 equiv.) was added, and stirred at room temperature for 10 minutes until DIPEA was completely dissolved. Then propargic acid (38.80 µL, 567.31 µmol, 1.5 equiv.) and HATU (172.57 mg, 453.85 µmol, 1.2 equiv.) were added to the reaction solution and stirred at room temperature for 6 hours. The reaction solution was diluted with 100 mL of dichloromethane, and washed with water and brine. The organic phases were dried over anhydrous Na₂SO₄. The organic solvent was removed under reduced pressure. The crude product was purified by silica gel column chromatography (dichloromethane/methanol = 60:1 to 30: 1) to obtain 135 mg of Compound 21, yellow solid, with a yield of 60%. ¹H NMR (400 MHz, DMSO-*d₆*) δ 10.09 (s, 1H), 8.95 (s, 1H), 8.73 (s, 1H), 8.06 (d, J = 2.9 Hz, 1H), 7.99 - 7.76 (m, 2H), 7.48 (dd, *J=* 9.1, 2.9 Hz, 1H), 5.90 - 5.77 (m, 1H), 4.14 (s, 1H), 3.32 - 3.07 (m, 8H), 2.98 (s, 2H), 2.60 (s, 4H), 2.43 (s, 3H), 2.31 (s, 3H), 2.29 - 2.16 (m, 2H), 1.97 - 1.83 (m, 2H), 1.83 - 1.70 (m, 2H), 1.66 - 1.53 (m, 2H). ESI-MS: *m*/*z* [M+H]⁺ calculated for C₃₁H₃₈N₉O₄⁺ 600.30, found 600.26.

### Synthesis of Compound 22

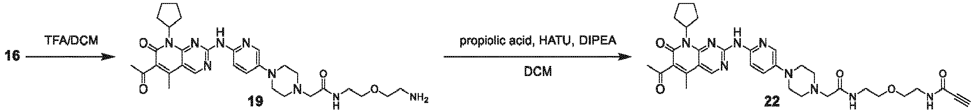

According to the synthesis method of Compound 21, Compound 16 (342 mg, 494.34 µmol, 1.0 equiv.) was used as a raw material to obtain 148 mg of Compound 22, yellow solid, with a yield of 47%. ¹H NMR (400 MHz, DMSO-*d₆*) δ 10.09 (s, 1H), 8.95 (s, 1H), 8.74 (t, 1H), 8.06 (d, *J =* 2.8 Hz, 1H), 7.85 (d, *J =* 9.0 Hz, 1H), 7.76 (t, *J* = 5.8 Hz, 1H), 7.47 (dd, *J =* 9.1, 2.9 Hz, 1H), 5.88 - 5.77 (m, 1H), 4.11 (s, 1H), 3.47 - 3.40 (m, 4H), 3.31 - 3.10 (m, 8H), 2.99 (s, 2H), 2.61 (s, 4H), 2.42 (s, 3H), 2.31 (s, 3H), 2.29 - 2.17 (m, 2H), 1.95 - 1.84 (m, 2H), 1.82 - 1.71 (m, 2H), 1.64 - 1.51 (m, 2H). ESI-MS: m/z [M+H]⁺ calculated for C₃₃H₄₂N₉O₅⁺ 644.33, found 644.29.

### Synthesis of Compound 23

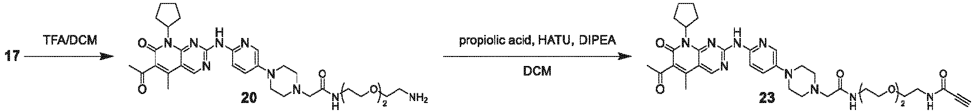

According to the synthesis method of Compound 21, Compound 17 (310 mg, 421.26 µmol, 1.0 equiv.) was used as a raw material to obtain 120 mg of Compound 23, yellow solid, with a yield of 41.5%. ¹H NMR (400 MHz, DMSO-*d₆*) δ 10.10 (s, 1H), 8.95 (s, 1H), 8.86 - 8.62 (m, 1H), 8.06 (d, *J*= 2.7 Hz, 1H), 7.85 (d, *J =* 9.0 Hz, 1H), 7.77 (t, *J =* 5.6 Hz, 1H), 7.48 (dd, *J* = 9.0, 2.7 Hz, 1H), 5.90 - 5.75 (m, 1H), 4.11 (s, 1H), 3.59 - 3.40 (m, 10H), 3.30 - 3.19 (m, 6H), 2.99 (s, 2H), 2.61 (s, 4H), 2.42 (s, 3H), 2.31 (s, 3H), 2.28 - 2.14 (m, 2H), 1.94 - 1.82 (m, 2H), 1.82 - 1.67 (m, 2H), 1.65 - 1.50 (m, 2H). ESI-MS: *m*/*z* [M+H]⁺ calculated for C₃₅H₄₆N₉O₆⁺ 688.36, found 688.20.

### Synthesis of Compound 24

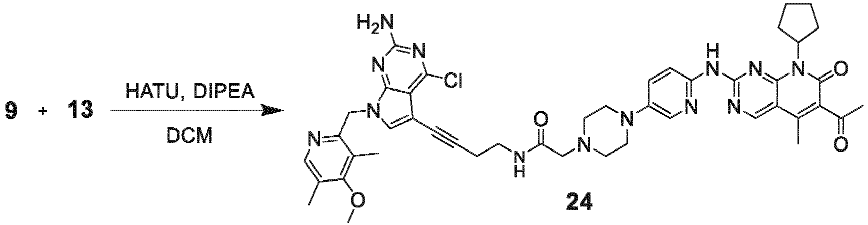

Compound 9 (100 mg, 259.83 µmol, 1.0 equiv.) and compound 13 (197.05 mg, 389.74 µmol, 1.5 equiv.) were dissolved in dry dichloromethane (15 mL). Then HATU (148.19 mg, 389.74 µmol, 1.5 equiv.) and DIPEA (226.29 µL, 1.30 mmol, 5.0 equiv.) were added to the reaction solution, and stirred overnight at room temperature. The reaction solution was diluted with 100 mL of dichloromethane, and washed with water and brine. The organic phases were dried over anhydrous Na₂SO₄. The organic solvent was removed under reduced pressure. The crude product was purified by silica gel column chromatography (dichloromethane/methanol = 50:1 to 20:1) to obtain 80 mg of Compound 24, yellow solid, with a yield of 35%. ¹H NMR (400 MHz, DMSO-*d₆*) δ 10.07 (s, 1H), 8.95 (s, 1H), 8.01 (s, 1H), 7.97 (d, *J =* 2.5 Hz, 1H), 7.93 (t, I = 5.6 Hz, 1H), 7.82 (d, *J =* 9.0 Hz, 1H), 7.33 (dd, *J =* 9.1, 2.4 Hz, 1H), 7.25 (s, 1H), 6.68 (s, 2H), 5.82 (p, *J* = 8.7 Hz, 1H), 5.24 (s, 2H), 3.70 (s, 3H), 3.41 - 3.34 (m, 2H), 3.13 (s, 4H), 3.00 (s, 2H), 2.68 - 2.54 (m, 6H), 2.43 (s, 3H), 2.31 (s, 3H), 2.29 - 2.18 (m, 5H), 2.13 (s, 3H), 1.93 - 1.82 (m, 2H), 1.81 - 1.71 (m, 2H), 1.64 - 1.51 (m, 2H). ¹³C NMR (100 MHz, DMSO) δ 202.92, 169.58, 163.78, 161.24, 160.00, 159.03, 158.72, 155.23, 153.87, 153.80, 152.07, 149.21, 144.76, 143.78, 142.55, 135.73, 131.49, 129.68, 125.50, 124.95, 123.85, 115.61, 107.96, 107.05, 95.52, 89.77, 74.82, 61.66, 60.26, 53.34, 53.06, 48.66, 46.86, 37.93, 31.78, 28.04, 25.58, 20.48, 14.10, 13.32, 10.69. ESI-HRMS: *m*/*z* [M+H]⁺ calculated for C₄₅H₅₁ClN₁₃O₄⁺ 872.3870, found 872.3872.

### Example 2: Preparation of Compound 25

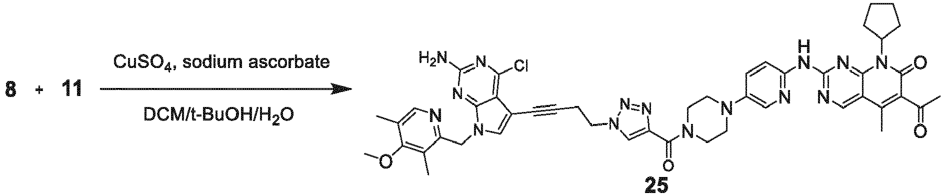

Compound 8 (123.36 mg, 300.26 µmol, 1.0 equiv.), compound 11 (150 mg, 300.26 µmol, 1.0 equiv.) and sodium ascorbate (237.93 mg, 1.20 mmol, 4.0 equiv.) were dissolved in t-BuOH/DCM (10 mL/5 mL). Then CuSO₄ (71.88 mg, 450.39 µmol, 1.5 equiv.) was dissolved in 5 mL of water and added dropwise to the above reaction solution. The reaction solution was heated to 50°C under the protection of N₂ and stirred for 3 hours. After the reaction solution was cooled to room temperature, the solvent was rotary dried. The residue was dissolved in 2M aqueous ammonia (20 mL), and extracted with dichloromethane. The organic layers were combined, and washed with water and brine. The organic phases were dried over anhydrous Na₂SO₄. The organic solvent was removed under reduced pressure. The crude product was purified by silica gel column chromatography (dichloromethane/methanol = 50:1 to 20:1) to obtain 110 mg of Compound 25, yellow solid, with a yield of 42%. ¹H NMR (400 MHz, DMSO-*d₆*) δ 10.15 (s, 1H), 8.96 (s, 1H), 8.67 (s, 1H), 8.10 (s, 1H), 8.05 (s, 1H), 7.90 (s, 1H), 7.52 (d, *J* = 7.7 Hz, 1H), 7.26 (s, 1H), 6.70 (s, 2H), 5.90 - 5.76 (m, 1H), 5.27 (s, 2H), 4.65 (t, *J =* 6.2 Hz, 2H), 4.25 (s, 2H), 3.81 (s, 2H), 3.71 (s, 3H), 3.23 (s, 4H), 3.12 (t, *J=* 6.1 Hz, 2H), 2.43 (s, 3H), 2.34 - 2.22 (m, 8H), 2.14 (s, 3H), 1.95 - 1.87 (m, 2H), 1.82 - 1.74 (m, 2H), 1.63 - 1.56 (m, 2H). ¹³C NMR (100 MHz, DMSO) δ 202.94, 163.79, 161.21, 160.01, 159.88, 153.84, 153.79, 152.11, 149.26, 143.30, 131.93, 129.42, 125.51, 123.84, 107.81, 94.97, 87.75, 75.89, 60.28, 53.42, 48.96, 46.88, 31.78, 28.04, 25.61, 21.13, 14.10, 13.33, 10.71. ESI-HRMS: *m*/*z* [M+H]⁺ calculated for C₄₆H₄₉ClN₁₅O₄⁺910.3775, found 910.3773.

### Example 3: Preparation of Compound 26

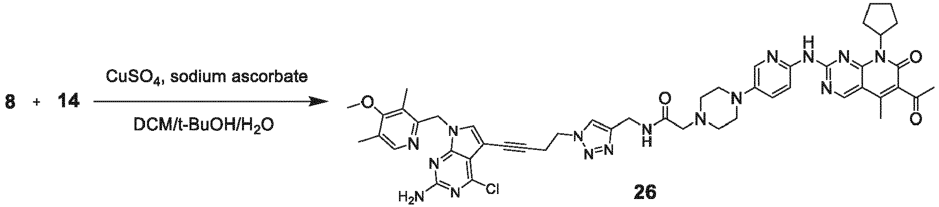

According to the synthesis method of Compound 25, Compound 8 (113.57 mg, 276.42 µmol, 1.0 equiv.) and Compound 14 (150 mg, 276.42 µmol, 1.0 equiv.) were used as raw materials to obtain 115 mg of Compound 25, yellow solid, with a yield of 43.6%. ¹H NMR (400 MHz, DMSO-*d₆*) δ 10.09 (s, 1H), 8.95 (s, 1H), 8.26 (t, *J* = 5.8 Hz, 1H), 8.04 (s, 2H), 7.99 (s, 1H), 7.84 (d, *J* = 9.0 Hz, 1H), 7.44 (d, *J* = 8.9 Hz, 1H), 7.26 (s, 1H), 6.69 (s, 2H), 5.88 - 5.77 (m, 1H), 5.26 (s, 2H), 4.55 (t, *J =* 6.6 Hz, 2H), 4.36 (d, *J =* 5.7 Hz, 2H), 3.71 (s, 3H), 3.17 (s, 4H), 3.10 - 2.89 (m, 4H), 2.57 (s, 4H), 2.42 (s, 3H), 2.38 - 2.18 (m, 8H), 2.14 (s, 3H), 1.93 - 1.83 (m, 2H), 1.81 - 1.71 (m, 2H), 1.62 - 1.53 (m, 2H). ¹³C NMR (100 MHz, DMSO) δ 202.93, 169.61, 163.79, 161.23, 160.01, 159.03, 158.73, 155.23, 153.86, 153.79, 152.14, 149.24, 145.37, 144.75, 143.84, 142.56, 135.79, 131.90, 129.68, 125.51, 125.13, 123.82, 123.29, 115.64, 107.85, 107.03, 95.08, 88.04, 75.73, 61.50, 60.28, 53.36, 53.04, 48.64, 46.88, 34.57, 31.78, 28.03, 26.82, 25.59, 21.36, 14.10, 13.33, 10.70. ESI-HRMS: m/z [M+H]⁺ calculated for C₄₈H₅₄C1N₁₆O₄⁺ 953.4197, found 953.4205.

### Example 4: Preparation of Compound 27

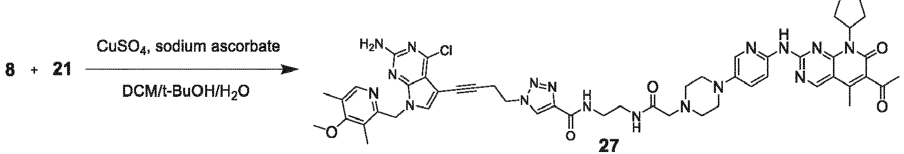

According to the synthesis method of Compound 25, Compound 8 (68.5 mg, 166.75 µmol, 1.0 equiv.) and Compound 21 (100 mg, 166.75 µmol, 1.0 equiv.) were used as raw materials to obtain 77 mg of Compound 27, yellow solid, with a yield of 45.7%. ¹H NMR (400 MHz, DMSO-*d₆*) δ 10.09 (s, 1H), 8.92 (s, 1H), 8.64 (s, 1H), 8.58 (t, *J* = 5.3 Hz, 1H), 8.04 (s, 1H), 8.00 (s, 1H), 7.95 - 7.78 (m, 2H), 7.42 (dd, *J* = 8.8, 1.7 Hz, 1H), 7.20 (s, 1H), 6.69 (s, 2H), 5.88 - 5.77 (m, 1H), 5.24 (s, 2H), 4.60 (t, *J* = 6.5 Hz, 2H), 3.71 (s, 3H), 3.48 - 3.35 (m, 4H), 3.16 - 3.03 (m, 6H), 2.96 (s, 2H), 2.55 (s, 4H), 2.43 (s, 3H), 2.30 - 2.20 (m, 8H), 2.15 (s, 3H), 1.94 - 1.85 (m, 2H), 1.83 - 1.73 (m, 2H), 1.63 - 1.54 (m, 2H). ¹³C NMR (100 MHz, DMSO) δ 202.95, 169.84, 163.79, 161.22, 160.50, 159.98, 158.99, 158.65, 155.20, 153.84, 153.74, 152.11, 149.24, 144.81, 143.78, 143.18, 142.55, 135.78, 131.82, 129.67, 127.00, 125.51, 125.13, 123.84, 115.50, 107.79, 107.01, 94.96, 87.61, 75.80, 65.49, 60.28, 53.40, 52.98, 48.99, 48.61, 46.86, 38.75, 38.62, 31.78, 28.04, 25.61, 21.13, 14.06, 13.33, 10.70. ESI-HRMS: *m*/*z* [M+H]⁺ calculated for C₅₀H₅₇ClN₁₇O₅⁺ 1010.4412, found 1010.4421.

### Example 5: Preparation of Compound 28

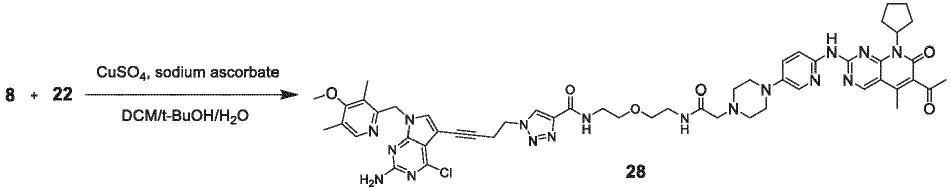

According to the synthesis method of Compound 25, Compound 8 (89.35 mg, 217.48 µmol, 1.0 equiv.) and Compound 22 (140 mg, 217.48 µmol, 1.0 equiv.) were used as raw materials to obtain 90 mg of Compound 28, yellow solid, with a yield of 39.2%. ¹H NMR (400 MHz, DMSO-*d₆*) δ 10.09 (s, 1H), 8.94 (s, 1H), 8.62 (s, 1H), 8.40 (t, *J* = 5.0 Hz, 1H), 8.05 (s, 2H), 7.86 (d, *J* = 8.3 Hz, 1H), 7.79 (s, 1H), 7.46 (d, *J* = 7.7 Hz, 1H), 7.23 (s, 1H), 6.69 (s, 2H), 5.88 - 5.75 (m, 1H), 5.25 (s, 2H), 4.61 (t, *J* = 6.0 Hz, 2H), 3.72 (s, 3H), 3.54 - 3.41 (m, 6H), 3.31 - 3.27 (m, 2H), 3.19 (s, 4H), 3.11 - 3.04 (m, 2H), 3.00 (s, 2H), 2.60 (s, 4H), 2.43 (s, 3H), 2.33 - 2.20 (m, 8H), 2.15 (s, 3H), 1.92 - 1.84 (m, 2H), 1.81 - 1.73 (m, 2H), 1.61 - 1.53 (m, 2H). ¹³C NMR (100 MHz, DMSO) δ 202.92, 167.43, 163.79, 161.23, 160.22, 159.99, 159.02, 158.69, 155.22, 153.84, 153.76, 152.13, 149.25, 144.76, 143.83, 143.14, 142.55, 135.82, 131.99, 129.13, 126.94, 125.50, 125.16, 123.84, 115.61, 107.82, 107.03, 94.98, 87.67, 75.80, 69.25, 69.06, 65.50, 60.28, 53.38, 52.99, 49.01, 48.63, 46.85, 38.74, 38.66, 31.77, 28.03, 25.58, 21.11, 14.07, 13.32, 10.70. ESI-HRMS: *m*/*z* [M+H]⁺ calculated for C₅₂H₆₁ClN₁₇O₆⁺ 1054.4674, found 1054.4683.

### Example 6: Preparation of Compound 29

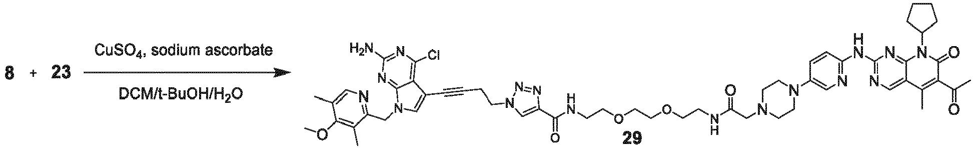

According to the synthesis method of Compound 25, Compound 8 (73.47 mg, 178.83 µmol, 1.0 equiv.) and Compound 23 (123 mg, 178.83 µmol, 1.0 equiv.) were used as raw materials to obtain 88 mg of Compound 29, yellow solid, with a yield of 44.8%. ¹H NMR (400 MHz, DMSO-*d₆*) δ 10.06 (s, 1H), 8.94 (s, 1H), 8.62 (s, 1H), 8.36 (t, *J* = 4.9 Hz, 1H), 8.05 (s, 2H), 7.85 (d, *J* = 8.8 Hz, 1H), 7.76 (s, 1H), 7.46 (d, *J* = 8.1 Hz, 1H), 7.23 (s, 1H), 6.67 (s, 2H), 5.88 - 5.74 (m, 1H), 5.26 (s, 2H), 4.61 (t, *J=* 6.0 Hz, 2H), 3.72 (s, 3H), 3.53 (s, 6H), 3.47 - 3.39 (m, 4H), 3.29 - 3.25 (m, 2H), 3.19 (s, 4H), 3.09 (t, *J*= 6.0 Hz, 2H), 2.99 (s, 2H), 2.60 (s, 4H), 2.42 (s, 3H), 2.34 - 2.19 (m, 8H), 2.15 (s, 3H), 1.96 - 1.84 (m, 2H), 1.83 - 1.72 (m, 2H), 1.64 - 1.53 (m, 2H). ¹³C NMR (100 MHz, DMSO) δ 202.89, 169.55, 163.81, 161.24, 160.19, 160.00, 159.03, 158.69, 155.23, 153.84, 153.78, 152.14, 149.27, 144.78, 143.84, 143.15, 142.55, 135.84, 131.84, 129.69, 126.93, 125.50, 125.16, 123.86, 115.62, 107.84, 107.05, 95.00, 87.69, 75.81, 70.02, 69.97, 69.48, 69.28, 61.57, 60.28, 53.40, 53.00, 49.02, 48.70, 46.87, 38.69, 38.61, 31.76, 28.03, 25.57, 21.13, 14.07, 13.32, 10.71. ESI-HRMS: *m*/*z* [M+H]⁺ calculated for C₅₄H₆₅ClN₁₇O₇⁺ 1098.4936, found 1098.4947.

### Example 7: Determination of binding affinity of compounds to HSP90α by surface plasmon resonance (SPR)

Biacore T200 control software was started to install CM5 chip according to the standard process, to prepare for the formal experiment. The buffer was used to flush the flow path system inside the whole system at a high flow rate. An appropriate program was selected according to the sample size. The trap chip was started, and the coupling buffer was PBS (pH 7.4). Sufficient sample, EDC/NHS, blocking buffer were prepared. The coupling program was started, and the final ligand coupling amount was about 12500 RU. After the coupling was completed, the assay was started. The buffer was changed to 1% DMSO PBS (pH 7.4). The analyte binding time was set to 120 s, with a flow rate of 30 µL/min. The dissociation time was set to 300 s, with a flow rate of 30 µL/min. The regeneration time was set to 30 s, with a flow rate of 30 µL/min. The corresponding samples to be tested were prepared according to the requirements, and the program was automatically run for testing. The results were analyzed. According to the operation results, data fitting analysis was carried out to obtain the final affinity fitting KD value.

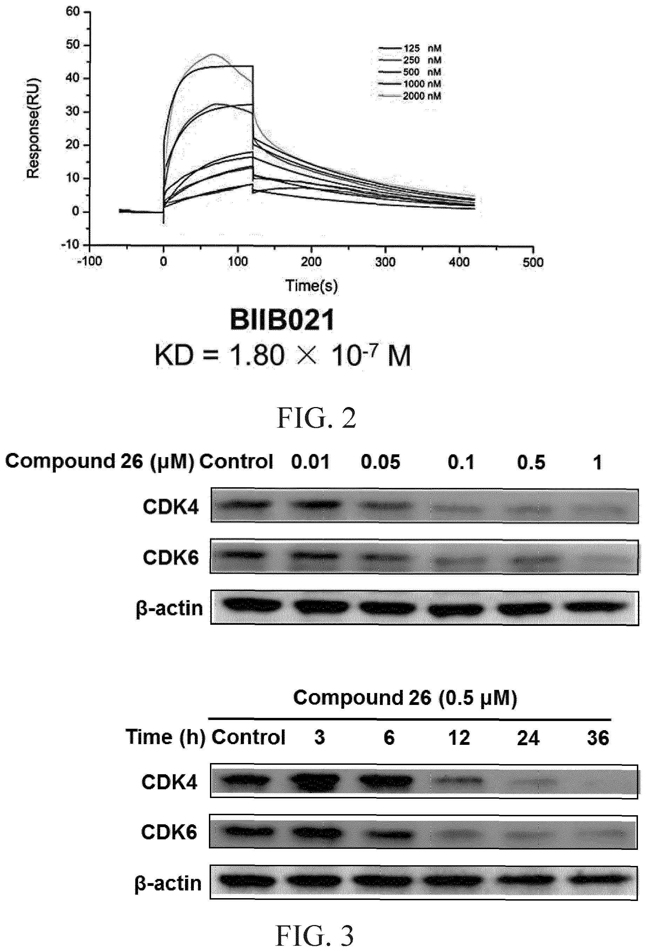

The results in FIG. 2 show that compared with HSP90 inhibitor BIIB021, the designed and synthesized small molecule HEMTACs have good affinity for HSP90, which indicates that the modification of N7 site of BIIB021 and the introduction of linker at this site have little impact on the binding of small molecule HEMTACs to HSP90, and proves the rationality of the previous design.

### Example 8: Western blot

After B16F10 cells were treated with the compound at different concentrations and for different periods of time, the levels of CDK4 and CDK6 proteins in the cells were measured by Western blot. Firstly, intracellular proteins were extracted and quantified. The cells were lysed with RIPA cell lysate (with a protease inhibitor), and proteins were collected, which were quantified by a BCA protein quantitative determination kit and quantified to the same concentration. Protein denaturation: The proteins were placed in a water bath at 100°C for 8 minutes to denature the proteins. Loading and gel electrophoresis: The loading amount per cell was generally 20 µL. The electrophoretic voltage of the upper concentration gel was 75 V and the electrophoretic voltage of the lower separation gel was 120 V When the protein marker of 40 KD reached about 1 cm at the end of the gel, the electrophoresis was ended. Membrane transfer: The membrane transfer was carried out using a wet transfer instrument, at a constant current of 250 mA for 90 minutes. The PVDF membrane should be soaked with methanol beforehand. Blocking: The PVDF membrane was placed in BSA blocking buffer for 1 h (with slow shaking in a shaker). Incubation with primary antibody: The primary antibody was diluted according to the instructions. The primary antibody was added, followed by incubation overnight at 4°C. Membrane washing: The membrane was washed with TBST washing buffer for 3 times, each time for 10 minutes. Incubation with secondary antibody: The second antibody was added, followed by incubation in a shaker at room temperature for 1 h. Membrane washing: The membrane was washed with TBST washing buffer for 3 times, each time for 10 minutes. Exposure: Equal volumes of ECL developing solutions A and B were mixed for exposure.

After B16F10 melanoma cells were treated with small molecule HEMTACs for 24 h, the Western blot results (FIG. 3) showed that CDK4/6 protein level decreased in a dose-dependent manner. Compound 26 had the highest degradation activity, which greatly enhanced the degradation activity of BIIB021. The DC₅₀ values (drug concentrations leading to 50% protein degradation) for Compound 26 inducing degradation of CDK4 and CDK6 in B16F10 cells were about 26 nM and 19 nM, respectively. In addition, the degradation of CDK4/6 was induced within 12 hours by treatment with 0.5 µM of Compound 26. The above results showed that a novel protein degradation method based on HSP90 inhibitor BIIB021 was successfully established.

### Example 9: Anti-tumor experiment in mice in vivo

B16F10 cells (10⁶ cells/mouse) were injected subcutaneously into the forelimb of C57BL/6 mice to establish an xenograft model of mouse B16F10 melanoma. When the tumor volume reached 100-200 mm³, the mice were randomly divided into four groups (n=6). The compound was injected intraperitoneally every day for two weeks, and the body weights and tumor volumes of the mice were measured every two days. The tumor length and width were measured along two orthogonal axes with a vernier caliper, and the tumor volume in the mouse was calculated based on an equation V (mm³)=(length ×width²)/2.

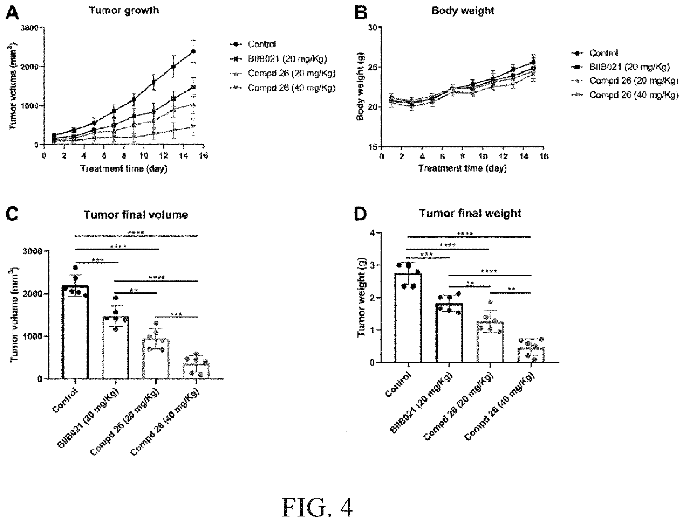

According to FIG. 4, Compound 26 (40 mg/kg) can significantly inhibit the growth of melanoma, and the anti-tumor effect of Compound 26 (20 mg/kg) is better than that of HSP90 inhibitor BIIB021 (20 mg/kg) in vivo. In addition, Compound 26 caused slight weight loss in mice, but showed no other signs of toxicity in all treatment groups. This experiment showed that the designed and synthesized small molecule HEMTAC 26 targeting CDK4/6 had an anti-melanoma effect in vivo.

The above embodiments are merely illustrative of the technical concept and features of the present invention, and provided for facilitating the understanding and practice of the present invention by those skilled in the art. However, the protection scope of the invention is not limited thereto. Equivalent variations or modifications made without departing from the spirit and essence of the present invention are intended to be contemplated within the protection scope of the present invention.

## Claims

1. A compound, having a structure of formula (I) below:
R₁-L-R₂ formula (I)
wherein:
(a) R1 is a ligand/conjugate of a targeted protein;
(b) R2 is an HSP90 ligand; and
(c) L is a linker through which R1 and R2 are linked.

2. The compound according to claim 1, wherein the targeted protein comprises kinases, a G protein-coupled receptor, a transcription factor, phosphatase, and a member of the RAS superfamily;
R1 is selected from compounds targeting the following: kinases, a G protein-coupled receptor, a transcription factor, phosphatase, and a member of the RAS superfamily; and
preferably, R1 is Palbociclib.

3. The compound according to claim 1, wherein R2 is a ligand BIIB021 of HSP90 or a derivative thereof; and
preferably, the general formula of the ligand BIIB021 of HSP90 or the derivative thereof is as follows:

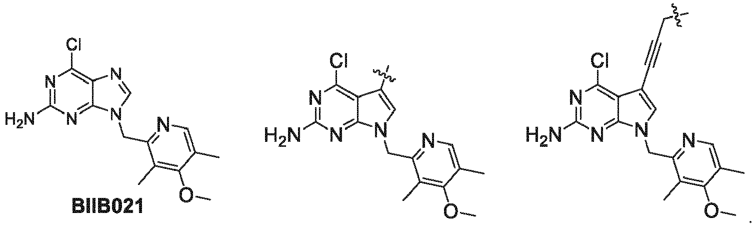

4. The compound according to claim 1, wherein the compound comprises any one or more of:

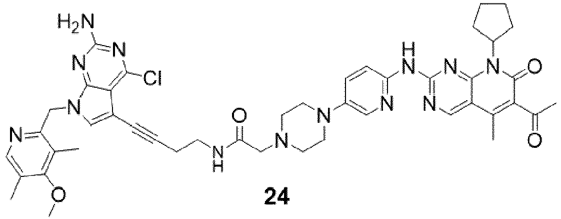

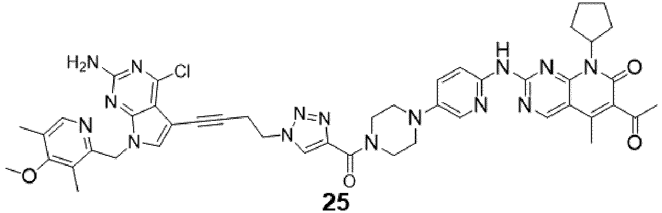

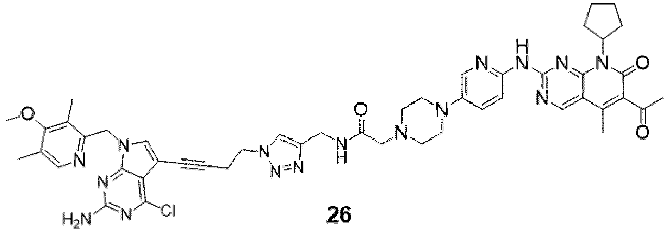

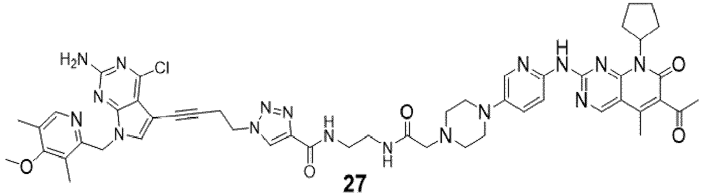

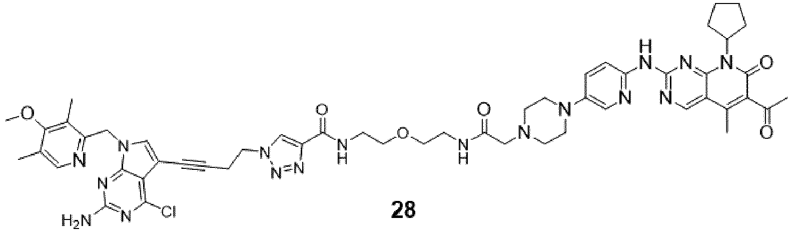

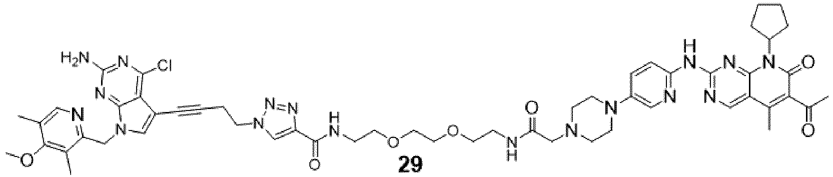

5. The compound according to any one of claims 1 to 4, wherein the compound further comprises pharmaceutically acceptable salts, stereoisomers, esters, prodrugs, solvates, and deuterated compounds thereof.

6. An application of the compound according to any one of claims 1 to 5 in preparing drugs for tumors, inflammation, and immune-related diseases,
wherein preferably, the diseases further comprise diseases associated with cell cycle dependent protein kinases 4 and 6.

7. A pharmaceutical composition, comprising the compound according to any one of claims 1 to 5.

8. A pharmaceutical preparation, comprising the compound according to any one of claims 1 to 5 and at least one pharmaceutically acceptable pharmaceutically inactive ingredient.

9. The pharmaceutical preparation according to claim 8, wherein the pharmaceutically inactive ingredient is a carrier, an excipient, or a diluent commonly used in pharmacy.

10. A method of treating tumors, inflammation, and immune-related diseases, the method comprising: administering to a subject a therapeutically effective amount of the compound according to any one of claims 1 to 4, the pharmaceutical composition according to claim 7, or the pharmaceutical preparation according to claim 8 or 9.
